# EUROPEAN PATENT APPLICATION

(11) **EP 4 342 317 A1**
(43) Date of publication of application: **27.03.2024**
(21) Application number: 23800301.6
(22) Date of filing: 01.06.2023
(51) Int. Cl.: A24F 40/60, A24F 40/50, A24F 40/42, G01R 19/25, G06N 3/08, G06Q 50/10, A24F 40/65, G02B 27/01, G06F 3/01, A24F 40/10

(54) **METHOD AND DEVICE FOR EMITTING SCENT CORRESPONDING TO CONTENT THROUGH CONTENT ANALYSIS**

(30) Priority: 11.08.2022 KR 20220100762
(71) Applicant: KT & G Corporation, Daejeon 34337 (KR)
(72) Inventor: KIM, Jae Hyun, Daejeon 34128 (KR); JEONG, Minseok, Daejeon 34128 (KR); CHUNG, Tae Young, Daejeon 34128 (KR)
(74) Representative: Ter Meer Steinmeister & Partner
(86) International application number: PCT/KR2023/007555
(87) International publication number: WO 2024/034800

(57) **Abstract**

A method of emitting fragrance corresponding to content through a content analysis is disclosed. A method of operating a display device includes recognizing a fragrance analysis target through analyzing content, extracting fragrance data corresponding to the fragrance analysis target, converting the fragrance data into a fragrance recipe based on cartridge information of a fragrance emitting device connected to the display device, and transmitting a control signal including the fragrance recipe to the fragrance emitting device.

## Description

The following description relates to a method of emitting fragrance corresponding to content through a content analysis, and specifically, to a method of emitting fragrance corresponding to content through an electronic device by analyzing content provided through a virtual display device.

### Background Art

Recently, computer graphics-applied virtual reality (VR), augmented reality (AR), and mixed reality (MR) technologies are in development. Here, VR technology refers to technology that uses a computer to build a virtual space that does not exist in the real world and then makes a user feel the virtual space like reality, and AR or MR technology refers to technology that adds computer-generated information to the real world to express, that is, technology that combines the real world and a virtual world to allow real-time interaction with a user.

Among these technologies, AR and MR technologies are utilized in conjunction with technologies in various fields. Even in the field of electronic cigarettes, demand for devices that implement VR or AR services is increasing, and a variety of research is being conducted accordingly.

### Disclosure of the Invention

### Technical Goals

Embodiments are to analyze content provided through a virtual display device to extract fragrance data corresponding to the content.

Embodiments are to convert fragrance data into a fragrance recipe and to emit a fragrance material corresponding to the fragrance recipe through an electronic device.

### Technical Solutions

According to an embodiment, a method of operating a display device includes recognizing a fragrance analysis target through analyzing content, extracting fragrance data corresponding to the fragrance analysis target, converting the fragrance data into a fragrance recipe based on cartridge information of a fragrance emitting device connected to the display device, and transmitting a control signal including the fragrance recipe to the fragrance emitting device.

The recognizing of the fragrance analysis target may include analyzing a scene by inputting the content to a pre-trained artificial neural network and recognizing the fragrance analysis target based on a result of analyzing the scene.

The recognizing of the fragrance analysis target may include receiving meta data corresponding to the content and recognizing the fragrance analysis target based on the meta data.

The fragrance emitting device may include a plurality of fragrance cartridges, and the cartridge information may include at least one of a number of the plurality of fragrance cartridges and types of the plurality of fragrance cartridges.

The converting may include mapping the plurality of predetermined ingredients to each of the plurality of fragrance cartridges based on the cartridge information and determining an amount of a fragrance material emitted from each of the plurality of fragrance cartridges based on concentration of each of the ingredients.

The control signal may further include information on an emitting time point of a fragrance material generated based on the fragrance recipe, and the information on the emitting time point is determined based on a time point at which the fragrance analysis target is provided in the content.

According to an embodiment, a method of operating an electronic device includes receiving a control signal including a fragrance recipe from a display device, mixing a plurality of fragrance materials according to the fragrance recipe, and providing an aerosolized fragrance material, wherein the fragrance recipe is obtained through a content analysis of the display device.

The electronic device may include a fragrance emitting device comprising a plurality of fragrance cartridges, and the fragrance recipe is determined based on a number of the plurality of fragrance cartridges and types of the plurality of fragrance cartridges.

The receiving of the control signal may include receiving of the control signal comprising information on an emitting time point of the fragrance material generated based on the fragrance recipe.

According to an embodiment, an electronic device includes a communication unit configured to receive a control signal including a fragrance recipe from a display device, and a fragrance emitting device configured to mix a plurality of fragrance materials according to the fragrance recipe and including a plurality of fragrance cartridges configured to provide an aerosolized fragrance material, wherein the fragrance recipe is obtained through a content analysis of the display device.

### Effects

Embodiments may provide analyzing content provided through a virtual display device to extract fragrance data corresponding to the content.

Embodiments may convert fragrance data into a fragrance recipe and may emit a fragrance material corresponding to the fragrance recipe through an electronic device.

### Brief Description of Drawings

FIG. 1 schematically illustrates a state in which a user smokes in virtual reality according to an embodiment.
FIG. 2 schematically illustrates a device for displaying a virtual image.
FIG. 3 is a flowchart illustrating an operating method of a display device according to an embodiment.
FIG. 4 schematically illustrates a configuration of an electronic device according to an embodiment.
FIG. 5 schematically illustrates a fragrance emitting cartridge and an aerosol generating device of an electronic cigarette device according to an embodiment.
FIG. 6 is a flowchart illustrating an operating method of an electronic device according to an embodiment.

### Best Mode for Carrying Out the Invention

The following detailed structural or functional description is provided as an embodiment only and various alterations and modifications may be made to embodiments. Here, embodiments are not construed as limited to the disclosure and should be understood to include all changes, equivalents, and replacements within the idea and the technical scope of the disclosure.

Although terms, such as first, second, and the like are used to describe various components, the components are not limited to the terms. These terms should be used only to distinguish one component from another component. For example, a first component may be referred to as a second component, and similarly the second component may also be referred to as the first component.

It should be noted that if it is described that one component is "connected", "coupled", or "joined" to another component, a third component may be "connected", "coupled", and "joined" between the first and second components, although the first component may be directly connected, coupled, or joined to the second component.

The singular forms "a", "an", and "the" are intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or populations thereof.

Unless otherwise defined, all terms used herein including technical or scientific terms have the same meaning as commonly understood by one of ordinary skill in the art to which embodiments belong. Terms, such as those defined in commonly used dictionaries, should be construed to have meanings matching with contextual meanings in the relevant art and the present disclosure, and are not to be construed as an ideal or excessively formal meaning unless otherwise defined herein.

Hereinafter, embodiments will be described in detail with reference to the accompanying drawings. When describing the embodiments with reference to the accompanying drawings, regardless of drawing numerals, like reference numerals refer to like elements and a repeated description related thereto will be omitted.

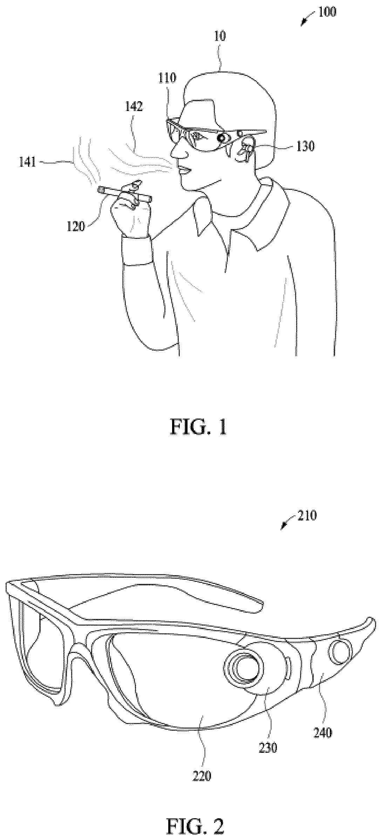

FIG. 1 schematically illustrates a state in which a user smokes in virtual reality according to an embodiment.

Referring to FIG. 1, a virtual smoking system 100 may include a device 110 for displaying a virtual image (hereinafter, referred to as a display device), an electronic device 120, and a playback device 130. A user 10 using the virtual smoking system 100 may wear the display device 110 and inhale or ingest an aerosol including a fragrance material through the electronic device 120.

In an embodiment, the electronic device 120 may have a general cigarette shape. In this case, the surface of an end portion of one side of the electronic device 120 may be coated with nicotine, or nicotine may be contained inside the end portion of one side of the electronic device 120. When the user 10 bites an end portion of one side of the electronic device 120 and inhales, the electronic device 120 may provide nicotine to the user 10. However, the shape of the electronic device 120 is not limited thereto.

In the present disclosure, the display device 110, the electronic device 120, and the playback device 130 included in the virtual smoking system 100 interoperate with each other, and thus, a more improved smoking experience may be provided to the user 10 who uses the virtual smoking system 100. A communication method among the display device 110, the electronic device 120, and the playback device 130 may include at least one of wireless local area network (WLAN) (wireless fidelity (Wi-Fi)), Bluetooth, ZigBee, Wi-Fi Direct (WFD), ultra-wideband (UWB), infrared data association (IrDA) communication, Bluetooth low energy (BLE), near-field communication (NFC), and Ant+. However, embodiments are not limited thereto.

The virtual smoking system 100 according to an embodiment may provide the user 10 with an olfactory effect as well as an audiovisual effect. More specifically, the user 10 may be provided with content through the display device 110 and at the same time, may be provided with a fragrance material through the electronic device 120. The fragrance material provided through the electronic device 120 may correspond to a fragrance analysis target present in the content provided through the display device 110. Content according to an embodiment may refer to data or information of signs, characters, figures, colors, voices, sounds, images, and videos (including complexes thereof).

As described in detail below, the display device 110 according to an embodiment may convert fragrance information into data through an analysis of a medium related to fragrance among content materials, may transmit the fragrance information converted into data to the electronic device 120, and when providing the content to the user 10 through the display device 110, may simultaneously provide a fragrance material related to the content through the electronic device 120.

FIG. 2 schematically illustrates a device for displaying a virtual image.

Referring to FIG. 2, an extended reality (XR) device 210 is illustrated as an example of the display device 110 according to an embodiment. The XR device 210 according to an embodiment may include a screen 220, a camera 230, and a temple portion 240. The XR device 210 may be one of devices for virtual reality (VR), augmented reality (AR), or metaverse, but the present embodiment is not limited thereto.

More specifically, when a user wears the XR device 210, the temple portion 240 of the XR device 210 may be seated on the ear of the user. However, the XR device 210 may include a band instead of the temple portion 240, and in this case, the band may be worn around the head of the user.

The XR device 210 according to an embodiment may provide the user with a VR, AR, or metaverse image, in which cigarette smoke is generated, through the screen 220. In an embodiment, the screen 220 may be controlled to be transparent or opaque depending on the purpose of use. When the XR device 210 provides a VR or metaverse image, the screen 220 may be controlled to be opaque, and when the XR device 210 provides an AR image, the screen 220 may be controlled to be transparent.

For example, the screen 220 may be formed of plastic such as polycarbonate or glass material but is not limited thereto. In addition, at least one coating method of anti-light reflection and anti-glare coating, anti-fog coating, or ultraviolet (UV)-protective coating may be applied to the screen 220.

When the user uses the XR device 210, the camera 230 according to an embodiment may scan a surrounding situation of reality to help the user use the XR device 210. In addition, the camera 230 may monitor a motion of the user and may transmit a control signal to the XR device 210.

An application may be installed on the XR device 210 according to an embodiment to provide various experiences to a user. For example, a VR smoking system may be provided by installing a smoking application to provide the user with a smoking experience close to reality.

Depending on embodiments, the XR device 210 may interoperate with an application installed on a user terminal (e.g., a smartphone) to provide a service corresponding to the application to the user. However, the present disclosure is not limited thereto.

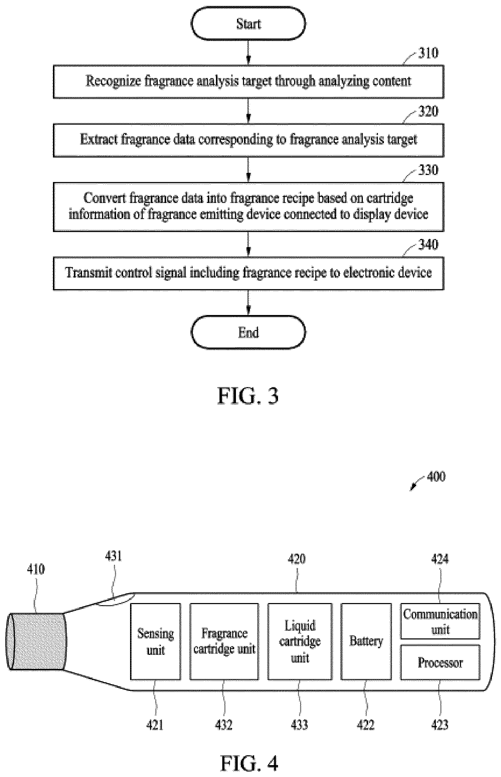

FIG. 3 is a flowchart illustrating an operating method of a display device according to an embodiment.

Referring to FIG. 3, operations 310 to 340 may be performed by the display device 110 described above with reference to FIGS. 1 and 2. Although operations of FIG. 3 may be performed in the illustrated order and manner, the order of some operations may change or some operations may be omitted, without departing from the spirit and scope of the illustrated embodiment. The operations illustrated in FIG. 3 may be performed in parallel or simultaneously.

In operation 310, the display device 110 according to an embodiment may recognize a fragrance analysis target through analyzing content. The fragrance analysis target according to an embodiment may refer to a medium related to fragrance among content materials. The fragrance analysis target according to an embodiment may be a particular object or a scene. For example, when a "rose" appears in the content, the "rose" may be the fragrance analysis target. Alternatively, when a "Cuba beach" scene appears in the content, the "Cuba beach" scene itself may be the fragrance analysis target. The fragrance analysis target according to an embodiment may be predefined, or may automatically recognize a medium related to fragrance among content materials even if not predefined.

A pre-trained artificial neural network may be installed in the display device 110 according to an embodiment. An artificial neural network according to an embodiment may be trained to receive content and to output the fragrance analysis target related to fragrance through a scene analysis. More specifically, the artificial neural network may be trained to minimize a difference between estimated fragrance analysis data and labeled fragrance analysis data corresponding to the content.

The display device 110 according to an embodiment may receive meta data corresponding to the content and may recognize the fragrance analysis target based on the meta data. For example, the meta data according to an embodiment may include fragrance analysis target information included in the corresponding content. The fragrance analysis target information according to an embodiment may include at least one of a list of fragrance analysis targets included in the content and time information when a corresponding fragrance analysis target appears in the content.

In operation 320, the display device 110 according to an embodiment may extract fragrance data corresponding to the fragrance analysis target. The fragrance data according to an embodiment may include fragrance components corresponding to the fragrance analysis target and concentration information of each of the fragrance components. The fragrance components according to an embodiment may be predetermined. For example, the fragrance data according to an embodiment may be expressed in predetermined fragrance components (e.g., "100" components).

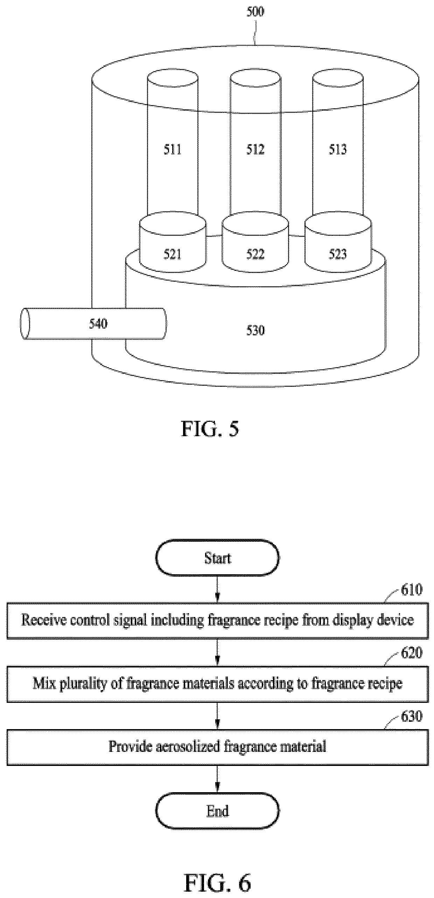

In operation 330, the display device 110 according to an embodiment may convert the fragrance data into a fragrance recipe based on cartridge information of a fragrance emitting device connected to the display device. As described in detail below, the electronic device 120 described with reference to FIG. 1 may include a fragrance emitting device. The fragrance emitting device according to an embodiment is a device for generating and providing a fragrance material, and may include a plurality of fragrance cartridges. The cartridge information according to an embodiment may include at least one of information on the number of the plurality of fragrance cartridges and information on types of the plurality of fragrance cartridges. A method of converting the fragrance data into the fragrance recipe according to an embodiment is described in detail with reference to FIGS. 4 and 5 below.

In operation 340, the display device 110 according to an embodiment may transmit a control signal including the fragrance recipe to the electronic device 120. The control signal according to an embodiment may be a signal for controlling the operation of the electronic device 120 connected to the display device 110.

The control signal according to an embodiment may further include information on an emitting time point of a fragrance material generated based on the fragrance recipe, and the information on the emitting time point according to an embodiment may be determined based on a point in time at which the fragrance analysis target is provided in the content. More specifically, the emitting time point may be determined identically to a point in time at which the content is provided, so that the user may be provided with the fragrance material through the fragrance emitting device of the electronic device 120 at the same time when the user is being provided with the content through the display device 110.

Depending on embodiments, the display device 110 or a server connected to the display device 110 may be a subject providing the virtual smoking system 100. The server according to an embodiment may function as a service platform that provides the virtual smoking system 100 to the user 10. The server according to an embodiment may provide a service through a dedicated application installed on the display device 110. In this case, the display device 110 may receive a control signal from the server and may operate under the control of the server. In other words, at least one of operations 310 to 340 may be performed by the server, and the display device 110 may operate under the control of the server. For example, the server may recognize the fragrance analysis target, may extract the fragrance data corresponding to the fragrance analysis target, and may perform converting of the fragrance recipe.

FIG. 4 schematically illustrates a configuration of an electronic device according to an embodiment.

The description provided with reference to FIGS. 1 to 3 may also apply to FIG. 4 and any repeated description related thereto will be omitted.

An electronic device 400 according to an embodiment may include a mouthpiece unit 410, a sensing unit 421, a battery 422, a processor 423, a communication unit 424, a fragrance sensor unit 431, a fragrance cartridge unit 432, and a liquid cartridge unit 433.

The sensing unit 421 according to an embodiment may sense a state of the electronic device 400 or a state around the electronic device 400 and may transmit information obtained by sensing to the processor 423.

The sensing unit 421 according to an embodiment may include at least one of a temperature sensor, an insertion detection sensor, or a puff sensor. However, embodiments are not limited thereto. For example, the sensing unit 421 may include the sensors described with reference to the drawings described above. The temperature sensor may sense the temperature and may transmit the temperature to the processor 423, so that a heat wire unit of the mouthpiece unit 410 may not be overheated.

The insertion detection sensor according to an embodiment may sense in the electronic device 400 whether the mouthpiece unit 410 is inserted and/or removed. The insertion detection sensor may include, for example, at least one of a film sensor, a pressure sensor, a light sensor, a resistive sensor, a capacitive sensor, an inductive sensor, and an infrared sensor, and may sense a signal change by the insertion and/or removal of the mouthpiece unit 410.

The sensing unit 421 according to an embodiment may further include at least one of a temperature/humidity sensor, an atmospheric pressure sensor, a magnetic sensor, an acceleration sensor, a gyroscope sensor, a position sensor (e.g., a global positioning system (GPS)), a proximity sensor, or a red, green, blue (RGB) sensor (e.g., an illuminance sensor), in addition to the sensors described above. A function of each sensor may be intuitively inferable from its name by one of ordinary skill in the art, and thus, a detailed description thereof is omitted herein.

The battery 422 may supply power to be used to operate the electronic device 400. The battery 422 may supply power to heat the liquid cartridge unit 433 or the fragrance cartridge unit 432. In addition, the battery 422 may supply power required for operations of the above-described other components included in the electronic device. The battery 422 may be a rechargeable battery or a disposable battery. The battery 422 may be, for example, a lithium polymer (Lipoly) battery.

The processor 423 according to an embodiment may control the overall operation of the electronic device 400. In an embodiment, the processor 423 may be implemented as an array of a plurality of logic gates, or may be implemented as a combination of a generalpurpose microprocessor and a memory in which a program executable by the microprocessor is stored. In addition, it is to be understood by one of ordinary skill in the art to which the present disclosure pertains that the processor 423 may be implemented in other types of hardware.

The communication unit 424 according to an embodiment may include at least one component for communicating with another electronic device. For example, the communication unit 424 may include a short-range wireless communication unit and a wireless communication unit.

The short-range wireless communication unit according to an embodiment may include a Bluetooth communication unit, a BLE communication unit, an NFC unit, a WLAN communication unit (e.g., Wi-Fi), a ZigBee communication unit, an IrDA communication unit, a WFD communication unit, a UWB communication unit, and an Ant+ communication unit. However, embodiments are not limited thereto.

The wireless communication unit according to an embodiment may include, for example, a cellular network communicator, an Internet communicator, a computer network (e.g., a local area network (LAN) or a wide area network (WAN)) communicator, or the like. However, embodiments are not limited thereto. The wireless communication unit may use subscriber information (e.g., international mobile subscriber identity (IMSI)) to identify and authenticate the electronic device 400 in a communication network.

The fragrance cartridge unit 432 according to an embodiment is described below with reference to FIG. 5.

The liquid cartridge unit 433 according to an embodiment may include a nicotine medium or a non-nicotine medium. The liquid cartridge unit 433 may vaporize actual liquid and may provide the liquid to the user. The electronic device according to an embodiment may vaporize a medium inside the liquid cartridge unit 433 and may provide the medium to the user. The nicotine medium may include materials (e.g., leaf tobacco, nicotine gel, nicotine activated carbon, nicotine-containing paraffin wax, tobacco medium compression bar, etc.) that may provide nicotine. The non-nicotine material may include health food such as red ginseng, caffeine, taurine, vitamins, etc. The electronic device may heat a material inside the liquid cartridge unit 433 to aerosolize the material, so that the user may inhale the aerosolized material. Heating methods may include various methods such as induction heating, external heating, internal heating, ultrasonic heating, and the like.

FIG. 5 schematically illustrates a fragrance emitting cartridge and an aerosol generating device of an electronic cigarette device according to an embodiment.

The description provided with reference to FIGS. 1 to 4 may also apply to the description provided with reference to FIG. 5, and any repeated description related thereto will be omitted.

A fragrance cartridge portion 500 according to an embodiment may include at least one fragrance cartridge, e.g., fragrance cartridges 511 to 513, fragrance material moving valves 521 to 523 attached to each cartridge, a fragrance aerosol generating portion 530 for aerosolizing a mixed solution, and a fragrance aerosol moving portion 540. In the present disclosure, the fragrance cartridge portion 500 is described as including three fragrance cartridges 511 to 513, but the number of fragrance cartridges is not limited to three, and the fragrance cartridge portion 500 may include various numbers of fragrance cartridges. Similarly, the number of solution moving valves 521 to 523 of the present disclosure is not also limited to three.

A processor of an electronic device according to an embodiment may control a fragrance cartridge portion based on a control signal received from a display device. The processor may control the operation of the fragrance cartridges 511 to 513, the fragrance material moving valves 521 to 523, the fragrance aerosol generating portion 530, and the fragrance aerosol moving portion 540 based on a fragrance recipe included in the control signal. The fragrance recipe may include mixing ratio data of fragrance materials included in the fragrance cartridges 511 to 513.

The fragrance cartridges 511 to 513 according to an embodiment may include fragrance materials that are different from each other. For example, the first fragrance cartridge 511 may include a light-based citrus fragrance material. The second fragrance cartridge 512 may include a green-based herbal-spicy fragrance material. The third fragrance cartridge 513 may include a heavy-based tobacco fragrance material.

The fragrance material moving valves 521 to 523 according to an embodiment may adjust a movement amount of the fragrance materials included in the fragrance cartridges 511 to 513 in response to the fragrance recipe, based on the control of the processor.

The fragrance aerosol generating portion 530 according to an embodiment may aerosolize a plurality of moved fragrance materials. The processor may control the fragrance aerosol generating portion 530 to aerosolize and spray a mixed solution included in the fragrance aerosol generating portion 530, when the user puffs for smoking.

The fragrance aerosol moving portion 540 according to an embodiment may be a tube through which a mixed fragrance material that is aerosolized is moved and sprayed. Since aerosolized fragrance needs to be sprayed according to the puff of the user, the processor may control the fragrance aerosol moving portion 540 so that the aerosolized fragrance according to the puff of the user may be sprayed.

FIG. 6 is a flowchart illustrating an operating method of an electronic device according to an embodiment.

Referring to FIG. 6, operations 610 to 630 may be performed by the electronic device 120 described with reference to FIGS. 1 to 5. The operations of FIG. 6 may be performed in the shown order and manner. However, the order of some operations may change or some operations may be omitted, without departing from the spirit and scope of the shown example. The operations shown in FIGS. 6 may be performed in parallel or simultaneously.

In operation 610, the electronic device 120 according to an embodiment may receive a control signal including a fragrance recipe from a display device.

In operation 620, the electronic device 120 according to an embodiment may mix a plurality of fragrance materials according to the fragrance recipe. The electronic device 120 according to an embodiment may transmit, to the fragrance cartridge portion 500, a control signal that includes the fragrance recipe and that is received from the display device 110. The fragrance cartridges 511 to 513 and the fragrance material moving valves 521 to 523 may move fragrance materials to the fragrance aerosol generating portion 530 according to the control signal to mix the plurality of fragrance materials. Here, the electronic device 120 may determine an amount of a plurality of fragrance materials emitted from each of a plurality of fragrance cartridges (e.g., an amount of fragrance materials moved from each of the fragrance cartridges 511 to 513) according to the fragrance recipe.

In operation 630, the electronic device 120 according to an embodiment may provide an aerosolized fragrance material. When the plurality of fragrance materials is mixed and aerosolized in the fragrance aerosol generating portion 530 according to an embodiment, the fragrance aerosol may be moved through the fragrance aerosol moving portion 540 and may be sprayed to the outside of the electronic device 120. Here, the electronic device 120 may control the fragrance cartridge portion 500, so that the fragrance aerosol may be sprayed according to the puff operation of the user.

The methods according to the embodiments may be recorded in non-transitory computer-readable media including program instructions to implement various operations of the embodiments. The media may also include, alone or in combination with the program instructions, data files, data structures, and the like. The program instructions recorded on the media may be those specially designed and constructed for the purposes of examples, or they may be of the kind well-known and available to one of ordinary skill in the computer software arts. Examples of non-transitory computer-readable media include magnetic media such as hard disks, floppy disks, and magnetic tape; optical media such as compact discread only memory (CD-ROM) and digital video discs (DVDs); magneto-optical media such as optical discs; and hardware devices that are specially configured to store and perform program instructions, such as read-only memory (ROM), random access memory (RAM), flash memory, and the like. Examples of program instructions include both machine code, such as produced by a compiler, and files containing higher level code that may be executed by the computer using an interpreter. The above-described devices may be configured to act as one or more software modules in order to perform the operations of the above-described embodiments, or vice versa.

The software may include a computer program, a piece of code, an instruction, or one or more combinations thereof, to independently or collectively instruct or configure the processing device to operate as desired. Software and/or data may be embodied permanently or temporarily in any type of machine, component, physical or virtual equipment, computer storage medium or device, or in a propagated signal wave capable of providing instructions or data to or being interpreted by the processing device. The software may also be distributed over network-coupled computer systems so that the software is stored and executed in a distributed fashion. The software and data may be stored by one or more non-transitory computer-readable recording mediums.

While the embodiments are described with reference to drawings, it will be apparent to one of ordinary skill in the art that various alterations and modifications in form and details may be made in these embodiments without departing from the spirit and scope of the claims and their equivalents. For example, suitable results may be achieved if the described techniques are performed in a different order, and/or if components in a described system, architecture, device, or circuit are combined in a different manner, or replaced or supplemented by other components or their equivalents.

Therefore, other implementations, other embodiments, and equivalents to the claims are also within the scope of the following claims.

## Claims

1. A method of operating a display device, the method comprising:
recognizing a fragrance analysis target through analyzing content;
extracting fragrance data corresponding to the fragrance analysis target;
converting the fragrance data into a fragrance recipe based on cartridge information of a fragrance emitting device connected to the display device; and
transmitting a control signal comprising the fragrance recipe to the fragrance emitting device.

2. The method of claim 1, wherein
the recognizing of the fragrance analysis target comprises:
analyzing a scene by inputting the content to a pre-trained artificial neural network; and
recognizing the fragrance analysis target based on a result of analyzing the scene.

3. The method of claim 1, wherein the recognizing of the fragrance analysis target comprises:
receiving meta data corresponding to the content; and
recognizing the fragrance analysis target based on the meta data.

4. The method of claim 1, wherein
the fragrance emitting device comprises a plurality of fragrance cartridges, and
the cartridge information comprises at least one of a number of the plurality of fragrance cartridges and types of the plurality of fragrance cartridges.

5. The method of claim 4, wherein
the converting comprises:
mapping the plurality of predetermined ingredients to each of the plurality of fragrance cartridges based on the cartridge information; and
determining an amount of a fragrance material emitted from each of the plurality of fragrance cartridges based on a concentration of each of the ingredients.

6. The method of claim 1, wherein
the control signal further comprises information on an emitting time point of a fragrance material generated based on the fragrance recipe, and
the information on the emitting time point is determined based on a time point at which the fragrance analysis target is provided in the content.

7. A method of operating an electronic device, the method comprising:
receiving a control signal comprising a fragrance recipe from a display device;
mixing a plurality of fragrance materials according to the fragrance recipe; and
providing an aerosolized fragrance material,
wherein the fragrance recipe is obtained through a content analysis of the display device.

8. The method of claim 7, wherein
the electronic device comprises a fragrance emitting device comprising a plurality of fragrance cartridges, and
the fragrance recipe is determined based on a number of the plurality of fragrance cartridges and types of the plurality of fragrance cartridges.

9. The method of claim 7, wherein
the receiving of the control signal comprises receiving the control signal comprising information on an emitting time point of the fragrance material generated based on the fragrance recipe.

10. A computer program combined with hardware and stored in a non-transitory computer-readable storage medium to perform the method of claim 1.

11. An electronic device comprising:
a communication unit configured to receive a control signal comprising a fragrance recipe from a display device; and
a fragrance emitting device comprising a plurality of fragrance cartridges configured to mix a plurality of fragrance materials according to the fragrance recipe and provide an aerosolized fragrance material,
wherein the fragrance recipe is obtained through a content analysis of the display device.
